# EUROPEAN PATENT APPLICATION

(11) **EP 2 893 877 A1**
(43) Date of publication of application: **15.07.2015**
(21) Application number: 14150657.6
(22) Date of filing: 09.01.2014
(51) Int. Cl.: A61B 5/08, A61B 5/0245, A61B 5/113, A61B 5/0452, G06F 19/00, A61B 5/04

(54) **Method and device for calculating a biosignal substitute from at least another biosignal of a patient**

(71) Applicant: Schmidt, Georg, 81675 München (DE)
(72) Inventor: Schmidt, Georg, 81675 München (DE)
(74) Representative: Grünecker Patent- und Rechtsanwälte PartG mbB

(57) **Abstract**

In order to satisfy a need to obtain the respiratory rate of a post-myocardial infarction patient with high reliability while minimizing the burden for the patient as well as the patient's possibility to manipulate the measurement results, the method comprises the following steps:
- Generating and/or storing at least two input data series based on recordings of said another biosignal of said patient
- Calculating multidimensional vectors on the basis of said input data series
- Calculating a variability of at least one parameter attributed to said multidimensional vectors over the time
- Producing at least one output data series indicating said variability of said parameter over the time.
- Deriving said biosignal substitute from at least one of said input data series and/or at least one of said output data series.

## Description

The invention relates to a method and a device for calculating a biosignal substitute from at least another biosignal of a patient such as a post-myocardial infarction patient.

As published in the European Heart Journal (2013) 34, 1644-1650, the respiratory rate (>= 18,6 breaths per minute (bpm)) can be used as a cardiac mortality risk predictor of a post-myocardial infarction patient. Like other cardiac mortality risk predictors such as LVEF (< 35%), Diabetes mellitus, GRACE score (>= 120 points) and presence of chronic obstructive pulmonary disease (COPD), the respiratory rate provides precise prediction results with respect to sudden cardiac mortality (univariable) and non-sudden cardiac mortality (univariable and multivariable) while it is comparably easy to measure.

Depending on the outcome of a mortality risk assessment, a specific medical treatment may be administered to the post-myocardial infarction patient. In view of the financial resources available for patients in statutory and private health insurance systems, there is an economical interest in administering specific and costly medical treatment predominantly to those post-myocardial infarction patients who can benefit most from such treatment.

The respiratory rate is typically monitored and measured in that the patient wears a chest belt under controlled conditions. However, the patient wearing the chest belt may feel discomfort and might be inclined to manipulate the respiratory rate measurement in the case where he or she knows about the medical significance of the measurement results, e.g. by taking deep breaths in order to slow down the respiratory rate. During such a recording, the patient may proactively affect the respiratory rate in this way, thereby reducing the medical significance of measurement results.

Therefore, there is a need to obtain the respiratory rate of a post-myocardial infarction patient with high reliability while minimizing the burden for the patient as well as the patient's possibility to manipulate the measurement results.

In order to solve the above identified problem, the invention provides the method for calculating a biosignal substitute from at least another biosignal of a patient such as a post-myocardial infarction patient according to claim 1, said method comprising the following steps:
- Generating and/or storing at least two input data series based on recordings of said another biosignal of said patient, wherein said recordings preferably originate from at least two different recording channels of a biosignal recorder.
- Calculating multidimensional vectors on the basis of said input data series, wherein each multidimensional vector is defined by at least two corresponding data points of different input data series.
- Calculating a variability of at least one parameter attributed to said multidimensional vectors over the time, wherein said at least one parameter is preferably an angle and/or a magnitude of a multidimensional vector.
- Producing at least one output data series indicating said variability of said parameter over the time.
- Deriving said biosignal substitute from at least one of said input data series and/or at least one of said output data series.

The invention has been conceived on the basis of the finding that the respiratory rate can be assessed with high precision and reliability from ECG recordings of a patient obtained through different recording channels of an ECG-recorder. In particular, it has been found that QRS-amplitudes and QRS two-dimensional vectors in pairs of ECG channel recordings ((x,y), (y,z), (x,z)) are indicative of the physiological variations of a patient when breathing. For example, variations of QRS-amplitudes and variations of an angle and/or a magnitude of a two-dimensional vector in a pair of ECG channel recordings ((x,y), (y,z), (x,z)) have been found to occur in response to an inclination of the cardiac axis in the patient's chest due to the expansion and contraction of the patient's lungs when breathing. The respiratory rate, in particular the nocturnal respiratory rate, identifies patients who fulfil the indication for primary ICD prophylaxis but who probably will not benefit from ICD therapy. Preferably, an ECG recorder with more than two recording channels (electrodes) will be used. The recording channels (electrodes) can be combined into a number of pairs. The output from each pair is referred to as a lead. Each lead looks at the heart from a different angle.

Preferably, said biosignal substitute substitutes at least one of the following biosignals or vital signs:
- Pulse rate or heart rate
- Blood pressure, preferably arterial blood pressure
- Respiratory rate

The term "biosignal" is meant to comprise any signal from biologic origin, in particular biosignals and vital signs.

Preferably, said another biosignal is at least one of the following biosignals:
- Electroencephalogram (EEG)
- Magnetoencephalogram (MEG)
- Galvanic skin response (GSR)
- Electrocardiogram (ECG), preferably an Electrocardiogram (ECG) recorded with at least two, three, four or more different recording channels, more preferably an Electrocardiogram (ECG) obtained through nocturnal continuous recordings under controlled conditions, preferably for at least six hours, more preferably between 0am and 6am.
- Mechanocardiogram (MCG)
- Electromyogram (EMG)

According to an embodiment of the invention, said step of generating and/or storing at least two input data series comprises at least one of the following sub-steps:
- Generating and/or storing a first input data series indicating the RR-intervals of subsequent QRS-complexes of an ECG-recording.
- Generating and/or storing a second input data series indicating the amplitudes of QRS-complexes recorded by a first ECG recording channel.
- Generating and/or storing a third input data series indicating the amplitudes of QRS-complexes recorded by a second ECG recording channel.
- Generating and/or storing a fourth input data series indicating the amplitudes of QRS-complexes recorded by a third ECG recording channel.

In a still further embodiment of the invention, said step of calculating multidimensional vectors on the basis of at least two of said input data series includes at least one of the following sub-steps:
- Attributing a data point value of an input data series to a corresponding data point value of a different input data series, so as to obtain the coordinates for each one of said multidimensional vectors in a virtual coordinate system, wherein preferably said corresponding data point values of said input data series have been recorded at the same time, preferably by different recording channels.
- Connecting said coordinates with the origin of said virtual coordinate system, so as to form each one of said multidimensional vectors in said virtual coordinate system.
- Repeating the preceding steps for at least two, preferably at least ten, more preferably at least twenty consecutive corresponding data point values of said input data series, so as to obtain a set of at least two, preferably at least ten, more preferably at least twenty consecutive multidimensional vectors on the basis of said input data series.

According to another embodiment of the invention, said step of calculating a variability of at least one parameter attributed to said multidimensional vectors over the time includes at least one of the sub-steps:
- Calculating an angle and/or a magnitude of each one of said multidimensional vectors using at least one of mathematical square, sin, cos, tan, arcsin, arccos and arctan-functions based on the coordinates of said multidimensional vectors in said virtual coordinate system.
- Repeating the preceding step for at least two, preferably at least ten, more preferably at least twenty consecutive multidimensional vectors obtained on the basis of said input data series.

According to still another embodiment of the invention, said step of producing said at least one output data series includes at least one of the following operations:
- Producing a first output data series indicating the angles of preferably at least twenty consecutive two-dimensional vectors defined by corresponding data points of first and second input data series over the time, wherein the angle is preferably the angle of the vector in relation to an abscissa and/or an ordinate in said virtual coordinate system.
- Producing a second output data series indicating the angles of preferably at least twenty consecutive two-dimensional vectors defined by corresponding data points of second and third input data series over the time, wherein the angle is preferably the angle of the vector in relation to an abscissa and/or an ordinate in said virtual coordinate system.
- Producing a third output data series indicating the angles of preferably at least twenty consecutive two-dimensional vectors defined by corresponding data points of first and third input data series over the time, wherein the angle is preferably the angle of the vector in relation to an abscissa and/or an ordinate in said virtual coordinate system.
- Producing a fourth output data series indicating the magnitudes of preferably at least twenty consecutive two-dimensional vectors defined by corresponding data points of first and second input data series over the time.
- Producing a fifth output data series indicating the magnitudes of preferably at least twenty consecutive two-dimensional vectors defined by corresponding data points of second and third input data series over the time.
- Producing a sixth output data series indicating the magnitudes of preferably at least twenty consecutive two-dimensional vectors defined by corresponding data points of first and third input data series over the time.

According to yet another embodiment of the invention, said step of deriving said biosignal substitute from said at least one input data series and/or at least one output data series includes at least one of the following sub-steps:
- Identifying preferably at least twenty consecutive periodic data points in at least two, preferably in all, of said input and/or output data series, said periodic data points being preferably local minima and/or maxima in each one of said input or output data series.
- Measuring the intervals between the consecutive periodic data points of the same kind in at least two, preferably in each one of said input and/or output data series.
- Averaging the intervals between the subsequent periodic data points in at least two, preferably in each one of said input and/or output data series.
- Calculating a respiratory rate substitute by using and/or inverting the average of the intervals between the subsequent periodic data points for said input and/or output data series.

The method according to the invention may be implemented by a computer with appropriate analysis software.

According to another independent aspect of the invention, the technical problem of the invention is solved by a device for calculating a biosignal substitute from at least another biosignal of a patient such as a post-myocardial infarction patient, said device being configured to perform the method according to at least one of the preceding embodiments.

Hence, all features attributed to the inventive method likewise apply to the inventive device.

Further preferred embodiments of the invention result from combinations of the features contained in the claims with other features contained in the description and the drawings.

### Brief description of the drawings

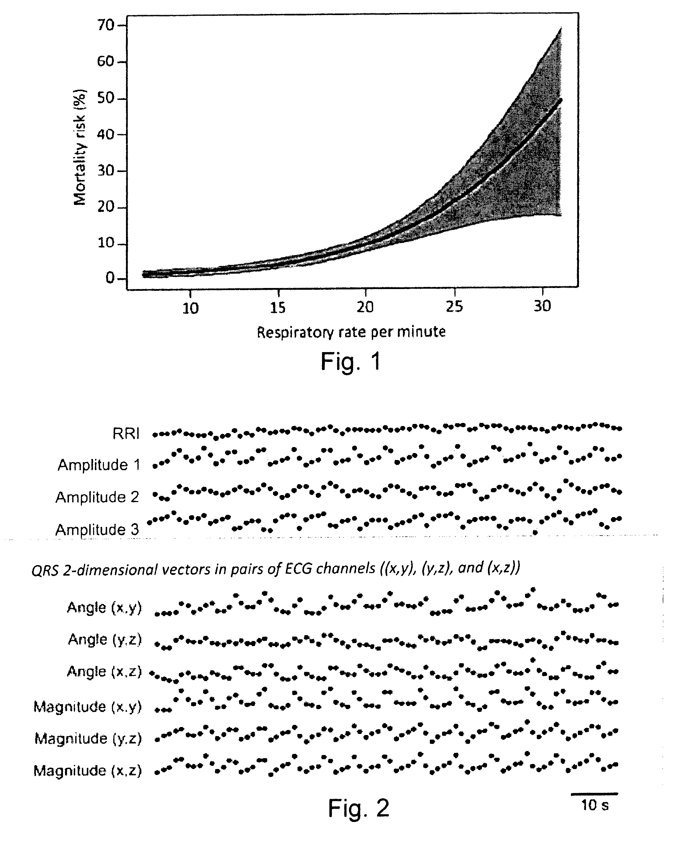

- Fig. 1: shows the effect of the respiratory rate on mortality, wherein the mortality risk is shown together with 95% confidence intervals, the respiratory rate being measured with a chest belt during 30-minutes monitoring under controlled conditions.
- Fig. 2: shows sections of input and output data series (time series 1 to 10) used as a basis for deriving the respiratory rate substitute according to the inventive method and device, wherein input data series referred to as *RRI* and *Amplitudes* 1, 2 and 3 are generated on the basis of nocturnal Holter-ECG recordings (0 am - 6 am) of a post-myocardial infarction patient and output data series referred to as *Angle (x,y), Angle (y,z), Angle (x,z), Magnitude (x,y), Magnitude (y,z)* and *Magnitude (x,z)* are QRS-two-dimensional vectors in pairs of ECG-channels ((x,y), (y,z) and (x,z)).

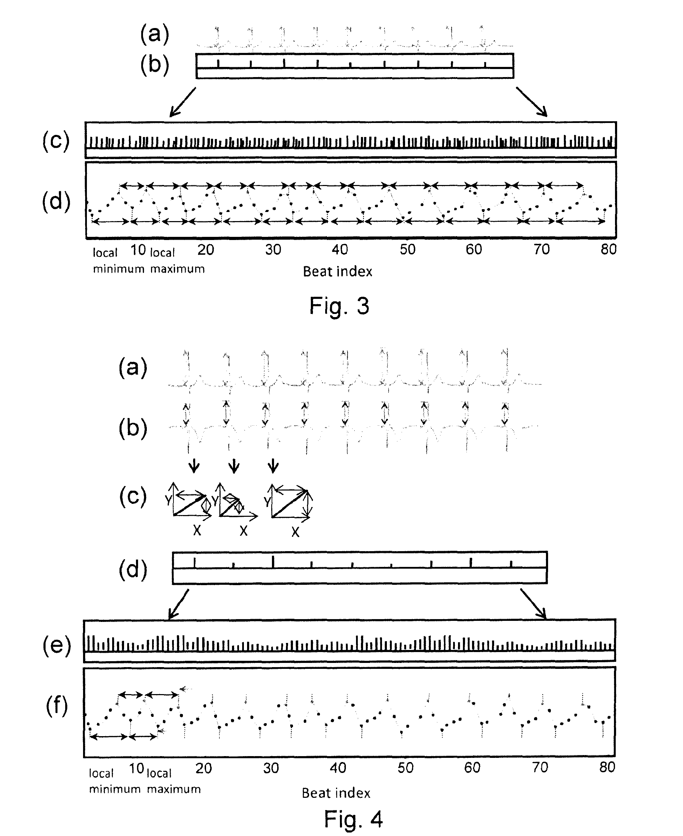

- Fig. 3: depicts the step of generating and/or storing an input data series indicating the amplitudes of QRS-complexes over the time for the recordings of one ECG recording channel, wherein Fig. 3 (a) indicates the amplitudes of QRS-complexes in a section of an ECG recording from one recording channel, Fig. 3 (b) depicts a bar chart indicating the absolute values of the amplitudes of QRS-complexes for subsequent heart beats in said section of said ECG recordings from one recording channel, Fig. 3 (c) depicts a bar chart indicating the absolute values of the amplitudes of QRS-complexes for subsequent heart beats in a larger section of said ECG recordings from one recording channel, and Fig. 3 (d) shows a graph depicting the absolute values of the amplitudes of QRS-complexes over the number of heart beats based on said ECG recordings from one recording channel.
- Fig. 4: depicts the step of calculating a magnitude of a two-dimensional vector and the step of producing one output data series indicating said variability of said magnitude of said two-dimensional vector over the time (the number of heart beats), wherein Figs. 4 (a) and (b) indicate the amplitudes of QRS-complexes in corresponding sections of ECG recordings from two different recording channels as contained in the input data series 2 and 3, Fig. 4 (c) depicts the sub-step of attributing an amplitude value (x) of a first input data series to a corresponding amplitude value (y) of a second input data series, so as to obtain the coordinates (x, y) for each one of said two-dimensional vectors in a virtual coordinate system, Fig. 4 (d) depicts the magnitude values of said two-dimensional vectors for said section of said ECG recordings, Fig. 4 (e) depicts depicts the magnitude values of said two-dimensional vectors for a larger section of said ECG recordings according to an output data series, and Fig. 4 (f) shows a graph depicting the magnitude values of said two-dimensional vectors over time (the number of heart beats) according to said output data series.

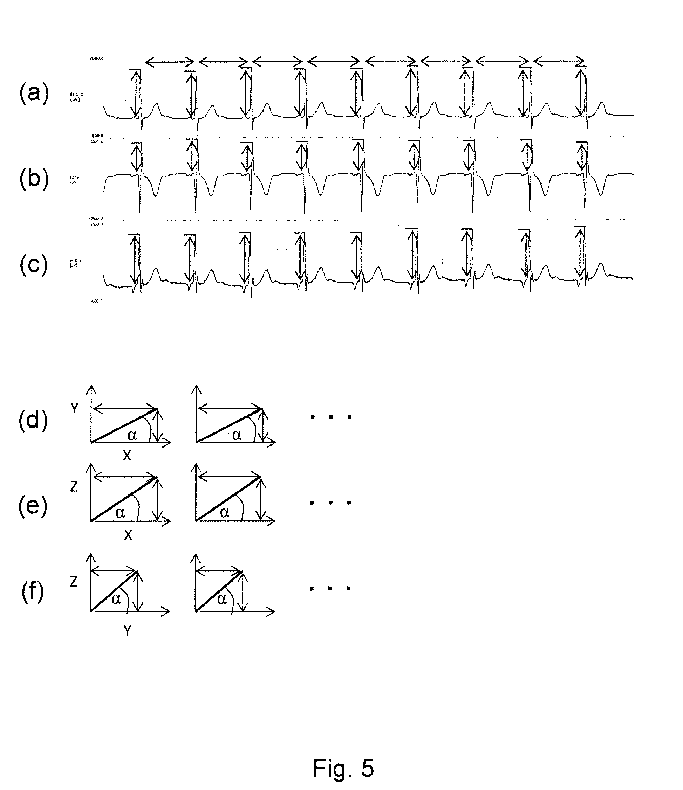

- Fig. 5: depicts the step of calculating the angles of two-dimensional vectors and the step of producing three output data series indicating a variability of said angles of said two-dimensional vectors over the time (the number of heart beats), wherein Figs. 5 (a), (b) and (c) indicate the amplitudes of QRS-complexes in corresponding sections of ECG recordings from three recording channels (x, y, z) as contained in the input data series 2, 3 and 4, and Figs. 5 (d), (e) and (f) depicts the sub-steps of attributing an amplitude value of one of said input data series to a corresponding amplitude value of another one of said input data series for each combination of input data series, so as to obtain the coordinates (x, y; y, z, x, z) of two-dimensional vectors in a virtual coordinate system, which are used for producing the output data series.

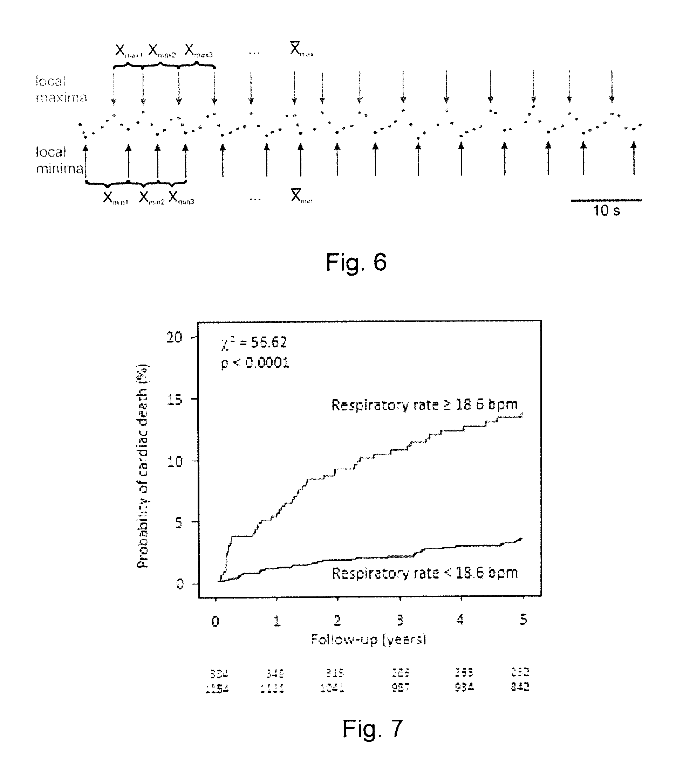

- Fig. 6: visualizes the mode of detecting of local minima and maxima in one of the input and output data series shown in Fig. 2, wherein the mean interval between preferably twenty consecutive local minima or maxima is used as a basis for deriving the respiratory rate substitute from ECG recordings of a post-myocardial infarction patient according to the inventive method and device.
- Fig. 7: depicts a diagram indicating the probability of cardiac death in percent over the first five follow-up years subsequent to a myocardial infarction, wherein a first (lower) graph indicates the probability of cardiac death of a post-myocardial infarction patient for which the respiratory rate has been measured or calculated to be less than 18.6 bpm, and wherein a second (upper) graph indicates the probability of cardiac death of a post-myocardial infarction patient for which the respiratory rate has been measured or calculated to be equal to or greater than 18.6 bpm.

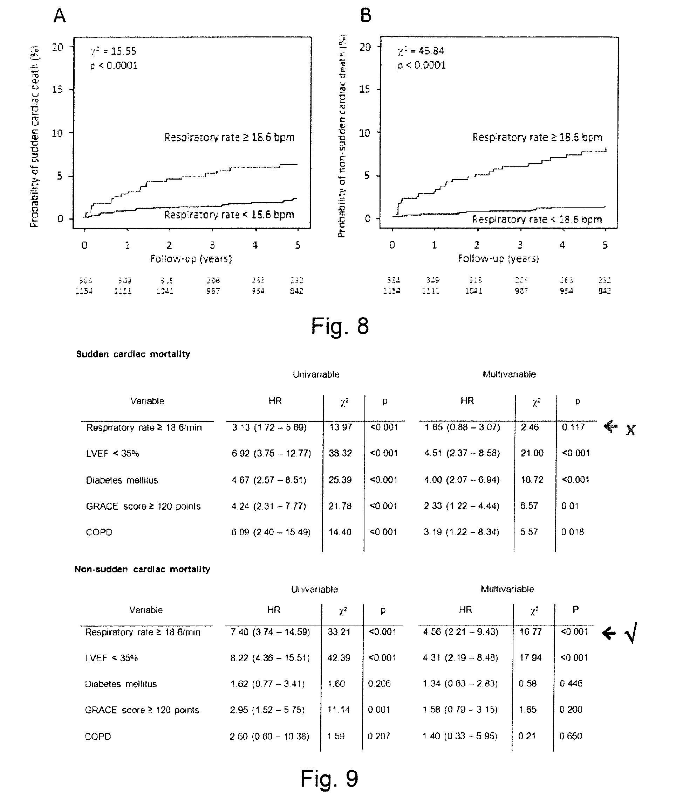

- Fig. 8: depicts diagrams indicating the probability of sudden cardiac death (A) and non-sudden cardiac death (B) in percent over the first five follow-up years subsequent to a myocardial infarction, wherein in both diagrams a respective first (lower) graph indicates the probability of cardiac death of a post-myocardial infarction patient for which the respiratory rate has been measured or calculated to be less than 18.6 bpm, and wherein a second (upper) graph indicates the probability of cardiac death of a post-myocardial infarction patient for which the respiratory rate has been measured or calculated to be equal to or greater than 18.6 bpm.
- Fig. 9: shows comparative tables containing the hazard ratios and predictive values of various mortality risk predictors for sudden cardiac mortality (top) and non-sudden cardiac mortality (bottom), both Univariable and Multivariable.

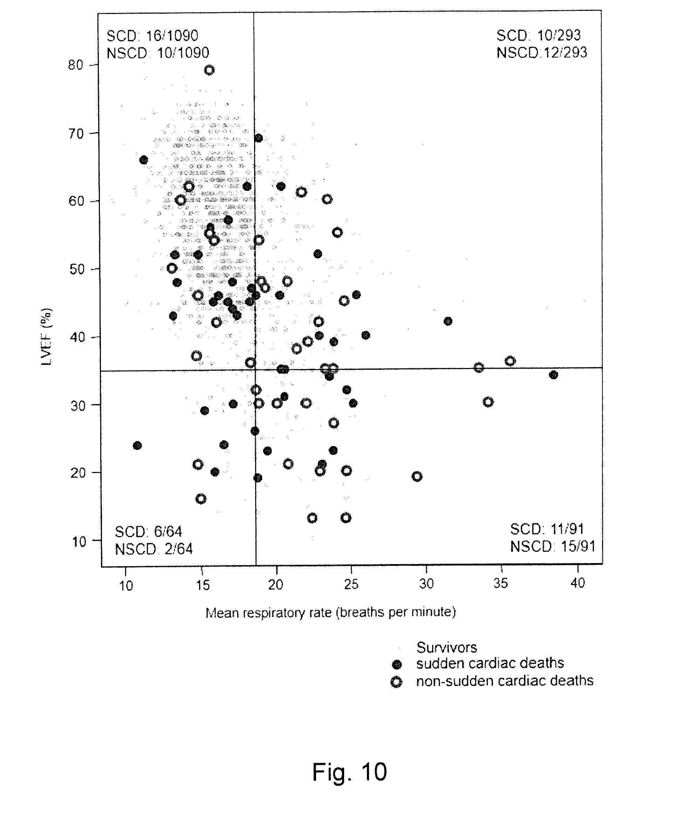

- Fig. 10: depicts a two-dimensional coordinate system displaying the LVEF (Left Ventricular Ejection Fraction) (in percent) over the mean respiratory rate (in breaths per minute) obtained for all individual survivors, sudden cardiac deaths (SCD) and non-sudden cardiac deaths (NSCD) of a study cohort, so as to visualize the relation between mortality risk for post-myocardial infarction patients and LVEF as well as the mean respiratory rate as mortality risk predictors, wherein the marked dots in the coordinate system indicate that the respiratory rate for a considerable fraction of the sudden cardiac deaths (SCD) and non-sudden cardiac deaths (NSCD) of the study cohort is equal to or greater than 18.6 bpm, as opposed to the vast majority of survivors.

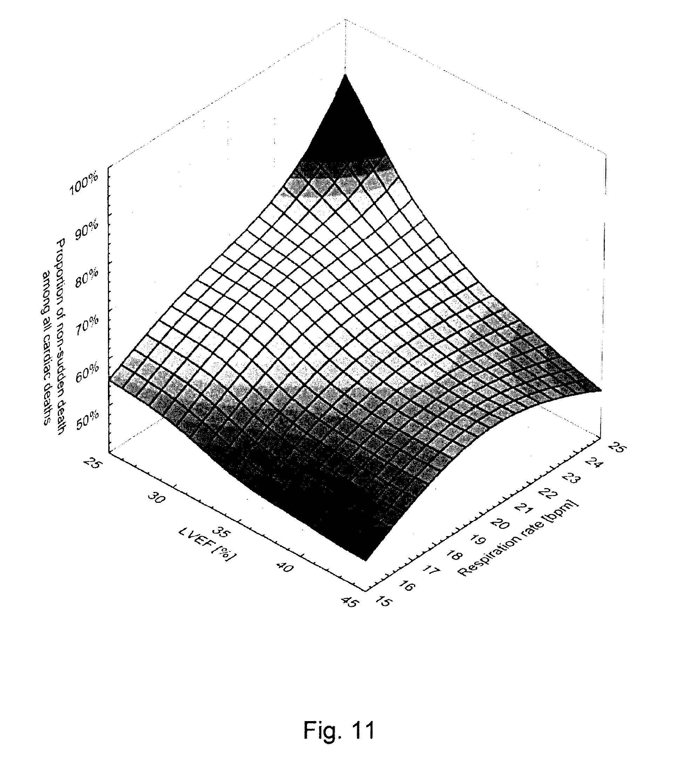

- Fig. 11: depicts a three-dimensional coordinate system indicating the proportion of non-sudden cardiac deaths over the LVEF score (in percent) and over the mean respiratory rate (in breaths per minute) based on the values obtained from the study cohort.

The preferred embodiment of the invention will be described in the following.

### Detailed description of the preferred embodiment

The preferred embodiment of the inventive method and device for calculating a biosignal substitute from at least another biosignal of a patient such as a post-myocardial infarction patient uses ECG recordings of said post-myocardial infarction patient obtained through three different recording channels (x), (y) and (z) of an ECG-recorder as input data series for calculating a respiratory rate substitute for said post-myocardial infarction patient. The ECG-recorder is preferably a stationary or Holter-ECG-recorder. The preferred recording time is between 0am and 6am. The inventive device is configured to perform the following individual steps of the inventive method as will be set out below.

The algorithm to detect the respiratory rate in an ECG signal is based on simultaneous ECG recordings, preferably for at least 30-minutes. It has been found that three groups of parameters from the ECG are influenced by respiratory activity: QRS amplitudes in individual ECG leads, QRS vectors between pairs of ECG leads, and RR intervals. The derivation of the respiratory rate from ECG recordings can be performed as follows:

### Step a: Generating and storing input data series

In step a, input data series originating from ECG recordings (e.g. 30-minutes nocturnal Holter-ECG recordings between 0 am - 6 am using an Oxford Excel Holter system, Oxford instruments; Pathfinder 700, Reynolds Medical; and Mortara Holter system, Mortara Instrument) of the post-myocardial infarction patient are generated and stored. As shown in Fig. 2, each one of the input data series contains a number of consecutive corresponding data points attributing respective recorded measurement values to respective measurement times. Since the data series have been simultaneously recorded through different recording channels, corresponding data points have been measured at the same measurement time and can be identified accordingly. In Figure 2, a representative example of all time series is shown. Since every data point corresponds to a QRS complex, the "sampling rate" of the signals is the heart rate. The main oscillation in all time series is caused by respiration.

In this embodiment, the following input data series are generated and stored:
- a first input data series indicating the RR-intervals of subsequent QRS-complexes of an ECG-recording (referred to as "RRI" in Fig. 2) as time series 1,
- a second input data series indicating the amplitudes of QRS-complexes recorded by a first ECG recording channel (referred to as "Amplitude 1" in Fig. 2) as time series 2,
- a third input data series indicating the amplitudes of QRS-complexes recorded by a second ECG recording channel (referred to as "Amplitude 2" in Fig. 2) as time series 3 and
- a fourth input data series indicating the amplitudes of QRS-complexes recorded by a third ECG recording channel (referred to as "Amplitude 3" in Fig. 2) as time series 4.

Figs. 3 (a) and (b) visualize how the amplitudes of QRS-complexes are derived from ECG recordings.

The input data series may be taken from different sources and biosignals, respectively. As an example, a first (group of) input data series may be based on ECG recordings indicating the amplitudes of QRS-complexes recorded by at least one ECG recording channel, wherein a second (group of) input data series may be based on blood pressure recordings indicating the systolic blood pressure.

### Step b: Calculating multidimensional vectors

Step b deals with calculating two-dimensional vectors on the basis of said input data series. In the present embodiment, each one of the two-dimensional vectors is defined by two corresponding data points of two different input data series originating from different ECG-recording channels (x), (y) and (z).

The two-dimensional vectors are calculated by attributing the data point values (xₙ, xₙ₊₁, ..) of an input data series (e.g. (x)) to the corresponding data point values (yₙ, yₙ₊₁, ..) of a different input data series (e.g. (y)), the corresponding data point values being recorded through different recording channels at the same time. This mathematical operation provides the coordinates (xₙ, yₙ; xₙ₊₁, yₙ₊₁; ..) for the two-dimensional vectors in a virtual coordinate system (x, y), as shown in Fig. 3 (c). The multidimensional vectors can be virtually formed in said virtual coordinate system by connecting said coordinates (xₙ, yₙ; xₙ₊₁, yₙ₊₁; ..) with the origin (0, 0) of said virtual coordinate system. The above procedure can be repeated as often as required, preferably until at least twenty consecutive multidimensional vectors have been calculated for at least three possible combinations ((x,y), (x,z), (y,z)) of said input data series.

The multidimensional vector can be defined by data points from different sources and biosignals, respectively. As an example, a first data point may represent the amplitude of a QRS-complexes and a second data point may represent the corresponding systolic blood pressure.

### Step c: Calculating a variability of at least one parameter of said multidimensional vectors

In step c, a variability of an angle and a magnitude of said multidimensional vectors over the time is calculated. The angle of the multidimensional vectors as shown in Figs. 5 (d), (e) and (f) may be calculated by a mathematical arctan-function (αₙ = arctan (yₙ/ xₙ); αₙ₊₁ = arctan (yₙ₊₁/ xₙ₊₁); ..), whereas the magnitude (m) of said multidimensional vectors as shown in Figs. 5 (d), (e) and (f) may be calculated by a mathematical square-function (mₙ² = xₙ² + yₙ²; mₙ₊₁² = x ₙ₊₁² + y ₙ₊₁²; ..). This sub-step is repeated for at least twenty consecutive multidimensional vectors for at least three possible combinations ((x,y), (x,z), (y,z)) of said input data series.

### Step d: Producing at least one output data function

Subsequent to the mathematical operations performed in step c, the following data series are produced:
- A first output data series (time series 5, referred to as "Angle (x, y)" in Fig. 2) indicating the angles of twenty consecutive two-dimensional vectors defined by corresponding data points of first (x) and second (y) input data series over the time.
- A second output data series (time series 6, referred to as "Angle (y, z)" in Fig. 2) indicating the angles of twenty consecutive two-dimensional vectors defined by corresponding data points of second (y) and third (z) input data series over the time.
- A third output data series (time series 7, referred to as "Angle (x, z)" in Fig. 2) indicating the angles of twenty consecutive two-dimensional vectors defined by corresponding data points of first (x) and third (z) input data series over the time.
- A fourth output data series (time series 8, referred to as "Magnitude (x, y)" in Fig. 2) indicating the magnitudes (m_{xy}) of twenty consecutive two-dimensional vectors defined by corresponding data points of first (x) and second (y) input data series over the time.
- A fifth output data series (time series 9, referred to as "Magnitude (y, z)" in Fig. 2) indicating the magnitudes (m_{yz}) of twenty consecutive two-dimensional vectors defined by corresponding data points of second (y) and third (z) input data series over the time.
- A sixth output data series (time series 10, referred to as "Magnitude (x, z)" in Fig. 2) indicating the magnitudes (m_{xz}) of twenty consecutive two-dimensional vectors defined by corresponding data points of first (x) and third (z) input data series over the time.

The input and output data series (time series 1 to 10) are shown in Fig. 2. Since every data point corresponds to a QRS complex, the "sampling rate" of the signals is the heart rate. The main oscillation in all time series is caused by respiration.

### Step e: Deriving said biosignal substitute

In step e, said respiratory rate substitute is derived from and calculated on the basis of the time series 1 to 10, i.e. the four input data series and the six output data series presented in Fig. 2. This step includes, for each one of the time series 1 to 10, the identification of twenty consecutive periodic data points such as local minima or local maxima and measuring the intervals between the consecutive periodic data points of the same kind according to the mode described in context with Fig. 6. Subsequently, the intervals between the twenty consecutive periodic data points will be averaged and a respiratory rate substitute will be calculated by using and/or inverting the average of the intervals obtained for each of said input or ouput data series. Preferably, the respiratory rate is calculated as the inverse of the median of all x⁻ max and x⁻min values from all time series.

As mentioned above, the invention is substantially based on the finding that the respiratory rate can be derived with high precision and reliability from multi-channel Holter-ECG recordings of a patient, wherein variations of QRS-amplitudes and variations of angles and/or magnitudes of QRS two-dimensional vectors in pairs of the ECG channel recordings ((x,y), (y,z), (x,z)) are indicative of the physiological variations of a patient when breathing. Therefore, the respiratory rate substitute obtained according to the inventive method and device clearly identifies patients who fulfil the indication for primary ICD prophylaxis but who probably will not benefit from ICD therapy. It has been proven in tests that there is no systematic bias in the measurement of ECG-derived respiratory rate.

## Claims

1. Method for calculating a biosignal substitute from at least another biosignal of a patient such as a post-myocardial infarction patient, **characterized by**
a. Generating and/or storing at least two input data series based on recordings of said another biosignal of said patient, wherein said recordings preferably originate from at least two different recording channels of a biosignal recorder.
b. Calculating multidimensional vectors on the basis of said input data series, wherein each multidimensional vector is defined by at least two corresponding data points of different input data series.
c. Calculating a variability of at least one parameter attributed to said multidimensional vectors over the time, wherein said at least one parameter is preferably an angle and/or a magnitude of a multidimensional vector.
d. Producing at least one output data series indicating said variability of said parameter over the time.
e. Deriving said biosignal substitute from at least one of said input data series and/or at least one of said output data series.

2. Method according to claim 1, **characterized by** said biosignal substitute substitutes at least one of the following biosignals or vital signs:
a. Pulse rate or heart rate
b. Blood pressure, preferably arterial blood pressure
c. Respiratory rate

3. Method according to at least one of the preceding claims, **characterized by** said another biosignal being at least one of the following biosignals:
a. Electroencephalogram (EEG)
b. Magnetoencephalogram (MEG)
c. Galvanic skin response (GSR)
d. Electrocardiogram (ECG), preferably an Electrocardiogram (ECG) recorded with at least two, three, four or more different recording channels, more preferably an Electrocardiogram (ECG) obtained through nocturnal continuous recordings under controlled conditions, preferably for at least six hours, more preferably between 0am and 6am.
e. Mechanocardiogram (MCG)
f. Electromyogram (EMG)

4. Method according to at least one of the preceding claims, **characterized by** said step of generating and/or storing at least two input data series comprises at least one of the following sub-steps:
a. Generating and/or storing a first input data series indicating the RR-intervals of subsequent QRS-complexes of an ECG-recording.
b. Generating and/or storing a second input data series indicating the amplitudes of QRS-complexes recorded by a first ECG recording channel.
c. Generating and/or storing a third input data series indicating the amplitudes of QRS-complexes recorded by a second ECG recording channel.
d. Generating and/or storing a fourth input data series indicating the amplitudes of QRS-complexes recorded by a third ECG recording channel.

5. Method according to at least one of the preceding claims, **characterized by** said step of calculating multidimensional vectors on the basis of at least two of said input data series includes at least one of the following sub-steps:
a. Attributing a data point value of an input data series to a corresponding data point value of a different input data series, so as to obtain the coordinates for each one of said multidimensional vectors in a virtual coordinate system, wherein preferably said corresponding data point values of said input data series have been recorded at the same time, preferably by different recording channels.
b. Connecting said coordinates with the origin of said virtual coordinate system, so as to form each one of said multidimensional vectors in said virtual coordinate system.
c. Repeating the preceding steps for at least two, preferably at least ten, more preferably at least twenty consecutive corresponding data point values of said input data series, so as to obtain a set of at least two, preferably at least ten, more preferably at least twenty consecutive multidimensional vectors on the basis of said input data series.

6. Method according to at least one of the preceding claims, **characterized by** said step of calculating a variability of at least one parameter attributed to said multidimensional vectors over the time includes at least one of the sub-steps:
a. Calculating an angle and/or a magnitude of each one of said multidimensional vectors using at least one of the following mathematical square, sin, cos, tan, arcsin, arccos and/or arctan-functions based on the coordinates of said multidimensional vectors in said virtual coordinate system.
b. Repeating the preceding step for at least two, preferably at least ten, more preferably at least twenty consecutive multidimensional vectors obtained on the basis of said input data series.

7. Method according to at least one of the preceding claims, **characterized by** said step of producing said at least one output data series includes at least one of the following operations:
a. Producing a first output data series indicating the angles of preferably at least twenty two-dimensional vectors defined by consecutive corresponding data points of first and second input data series over the time, wherein the angle is preferably the angle of the vector in relation to an abscissa and/or an ordinate in said virtual coordinate system.
b. Producing a second output data series indicating the angles of preferably at least twenty two-dimensional vectors defined by consecutive corresponding data points of second and third input data series over the time, wherein the angle is preferably the angle of the vector in relation to an abscissa and/or an ordinate in said virtual coordinate system.
c. Producing a third output data series indicating the angles of preferably at least twenty two-dimensional vectors defined by consecutive corresponding data points of first and third input data series over the time, wherein the angle is preferably the angle of the vector in relation to an abscissa and/or an ordinate in said virtual coordinate system.
d. Producing a fourth output data series indicating the magnitudes of preferably at least twenty two-dimensional vectors defined by consecutive corresponding data points of first and second input data series over the time.
e. Producing a fifth output data series indicating the magnitudes of preferably at least twenty two-dimensional vectors defined by consecutive corresponding data points of second and third input data series over the time.
f. Producing a sixth output data series indicating the magnitudes of preferably at least twenty two-dimensional vectors defined by consecutive corresponding data points of first and third input data series over the time.

8. Method according to at least one of the preceding claims, **characterized by** said step of deriving said biosignal substitute from said at least one input data series and/or at least one output data series includes at least one of the following sub-steps:
a. Identifying preferably at least twenty consecutive periodic data points in at least two, preferably in all, of said input or output data series, said periodic data points being preferably local minima and/or maxima in each one of said input and/or output data series.
b. Measuring the intervals between the consecutive periodic data points of the same kind in at least two, preferably in each one of said input and/or output data series.
c. Averaging the intervals between the subsequent periodic data points measured in at least two, preferably in each one of said input and/or output data series.
d. Calculating a respiratory rate substitute by using and/or inverting the averages of the intervals between the subsequent periodic data points measured for each one said input or output data series.

9. Device for calculating a biosignal substitute from at least another biosignal of a patient such as a post-myocardial infarction patient, said device being configured to perform the method according to at least one of the preceding claims.
